(19) Europäisches Patentamt
European Patent Office
Office européen des brevets

(11) Veröffentlichungsnummer: **0 080 658**
**B1**

(12) # EUROPÄISCHE PATENTSCHRIFT

(45) Veröffentlichungstag der Patentschrift: **30.09.87**

(21) Anmeldenummer: **82110635.8**

(22) Anmeldetag: **18.11.82**

(51) Int. Cl.[4]: **C 07 C 29/09,** C 07 C 35/14, C 07 C 35/06, C 07 C 35/20, C 07 D 317/20, C 07 D 317/46, C 07 C 29/128

(54) **Verfahren zur Herstellung von cyclischen 1,2-cis-Diolen aus cyclischen 1,2-Epoxiden.**

(30) Priorität: **02.12.81 DE 3147737**

(43) Veröffentlichungstag der Anmeldung:
**08.06.83 Patentblatt 83/23**

(45) Bekanntmachung des Hinweises auf die Patenterteilung:
**30.09.87 Patentblatt 87/40**

(84) Benannte Vertragsstaaten:
**BE CH DE FR GB IT LI NL**

(56) Entgegenhaltungen:
**EP-A-0 001 082**
**DE-A-1 793 247**
**DE-B-1 096 348**
**DE-B-1 169 459**
**FR-A-2 429 196**
**GB-A-2 011 401**
**GB-A-2 011 402**
**US-A-2 773 070**
**US-A-4 187 373**

**Die Akte enthält technische Angaben, die nach dem Eingang der Anmeldung eingereicht wurden und die nicht in dieser Patentschrift enthalten sind.**

(73) Patentinhaber: **BAYER AG**
**Konzernverwaltung RP Patentabteilung**
**D-5090 Leverkusen 1 Bayerwerk (DE)**

(72) Erfinder: **Kölbl, Heinz, Dr.**
**Andreas-Gryphius-Strasse 20**
**D-5000 Köln 80 (DE)**

(56) Entgegenhaltungen:
**CHEMICAL ABSTRACTS, Band 58, Nr. 11, 27. Mai 1963, Spalten 11208h-11209a, Columbus, Ohio, USA, S.Z. LEVIN et al.: "Synthesis of alkylene carbonates (ethylene carbonates)"**

**CHEMICAL ABSTRACTS, Band 96, Nr. 5, März 1982, Seite 553, Nr. 68320s, Columbus, Ohio, USA**

**CHEMICAL ABSTRACTS, Band 96, Nr. 5, März 1982, Seite 553, Nr. 68319y, Columbus, Ohio, USA**

**JOURNAL OF THE CHEMICAL SOCIETY, Physical Organic Chemistry, Teil B, 1971, Seiten 1035-1040, London, G.B., J. KATHHENDLER et al.: "Organic carbonates. Part XI. Conformational and other effects of *substitution in the acid-catalysed hydrolysis of* highly branched ethylene and trimethylene carbonates"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft ein neues, zweistufiges Verfahren zur Herstellung von cyclischen 1,2-*cis*-Diolen aus cyclischen 1,2-Epoxiden; als Zwischenprodukte werden dabei die entsprechenden cyclischen 1,2-*cis*-Carbonate gebildet Die cyclischen 1,2-*cis*-Diole können ihrerseits als Zwischenprodukte zur Herstellung herbizider Wirkstoffe verwendet werden.

Es ist bereits bekannt, daß man cyclische 1,2-*cis*-Diole z.B. aus Cycloalkenen durch Umsetzung mit Kaliumpermanganat, Thalliumacetat oder Osmiumtetroxid herstellen kann (vgl. hierzu z.B. Organic Synthesis *59,* 169—176 (1980)). Auch durch Cyclisierung von Dicarbonsäureestern mit Natrium/Chlortrimethylsilan und anschließende Hydrierung lassen sich cyclische 1,2-*cis*-Diole gewinnen (vgl. DE—OS 2 163 394). Weiterhin ist bekannt, daß man cyclische 1,2-*cis*-Diole aus Benzanthracenen durch Umsetzung mit Vinylcarbonat unter Stickstoff und auschließender Hydrolose der cyclischen Benzanthracencarbonate erhält (vgl. US—P 4 187 373). Diese Verfahren sind jedoch mit Nachteilen behaftet, wie z.B. unzureichende Ausbeuten und Selektivitäten, schwierige technische Durchführbarkeit oder Umgang mit sehr toxischen oder teuren Hilfsstoffen.

Es wurde nun gefunden, daß man cyclische 1,2-*cis*-Diole der allgemeinen Formel I

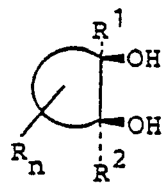

(I)

worin der carbocyclische Ring 5—7 Kohlenstoffatome enthält und

$R^1$ und $R^2$ gleich oder verschieden sind und unabhängig voneinander für Wasserstoff oder gegebenenfalls substituierte Alkyl-, Cycloalkyl-, Alkenyl-, Aryl- oder Aralkylrest stehen,

n für 1, 2, 3 oder 4 steht und

R für Wasserstoff, Hydroxy, Halogen, Cyano oder gegebenenfalls substituierte Alkyl-, Cycloalkyl, Alkenyl-, Aryl-, Aralkyl-, Alkoxy-, Alkoxycarbonyl-, Aryloxy-, Alkylthio-, Arylthio- oder heterocyclische Reste steht, wobei für den Fall n >1 die Reste R gleich oder verschieden sein können und wobei 2 benachbarte Reste R, gegebenenfalls auch zweimal, für eine Doppelbindung stehen können, mit hoher Ausbeute und Selektivität erhält, wenn man cyclische 1,2-Epoxide der allgemeinen Formel II

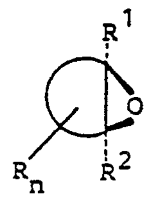

(II)

worin der carbocyclische Ring 5—7 Kohlenstoffatome enthält und $R^1$, $R^2$, n und R die oben angegebene Bedeutung haben,

mit Kohlendioxid in Gegenwart eines Carbonierungskatalysators bei Drucken von 20—200 bar und bei Temperaturen zwischen 100 und 300°C umsetzt, wobei der Druck durch Nachpressen von Kohlendioxid solange konstant gehalten wird, bis keine Druckabnahme mehr beobachtet wird, (1. Stufe) und die hierbei gebildeten cyclischen 1,2-*cis*-Carbonate der allgemeinen Formel III

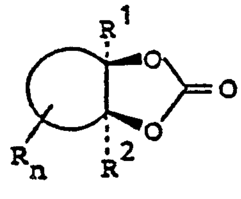

(III)

worin der carbocyclische Ring wiederum 5—7 Kohlenstoffatome enthält und $R^1$, $R^2$, n und R die oben angegebene Bedeutung haben,

mit Wasser, wäßrigen Salzlösungen, verdünnten wäßrigen Säuren oder Basen hydrolysiert oder mit der Lösung eines Alkoholats in einem Alkohol solvolysiert, jeweils bei erhöhter Temperatur und gegebenenfalls unter erhöhtem Druck (2. Stufe).

Es konnte durch unabhängige Synthese gezeigt werden, daß bei der 1. Stufe des erfindungsgemäßen Verfahren überraschenderweise die *cis*-Carbonate (III) in reiner Form, ohne Verunreinigung durch die trans-Verbindungen, entstehen. In früheren Patentschriften (z.B. US—PS 2 773 070) wurde zwar die

Herstellung von Cyclohexencarbonat in mäßiger Ausbeute und Selektivität durch katalysierte Umsetzung von Cyclohexenoxid mit $CO_2$ bei erhöhtem Druck und erhöhter Temperatur beschrieben, es wurde jedoch keine Aussage über die stereochemische Zusammensetzung des Produkts gemacht.

Die *cis*-Carbonate (III) können mit Wasser, wäßrigen Salzlösungen oder verdünnten wäßrigen Säuren oder Basen zu den 1,2-*cis*-Diolen (I) hydrolysiert werden; sie können aber auch durch Umsetzung mit Alkoholaten in einem Alkohol unter Bildung der 1,2-*cis*-Diole (I) solvolysiert werden. Die letztere Reaktion zeigt an, daß die Bildung des Diols bewirkende Nukleophil ausschließlich am Carbonyl-C-Atom des Carbonats — nicht aber an $C^1$ oder $C^2$ des carbocyclischen Ringes — angreift und so die Stereochemie erhalten bleibt.

Setzt man als Ausgangsprodukt 1,2-Dimethyl-cyclohexen-(1,2)-oxid ein, so kann der Reaktionsverlauf durch das folgende Formelschema wiedergegeben werden:

1. Stufe:

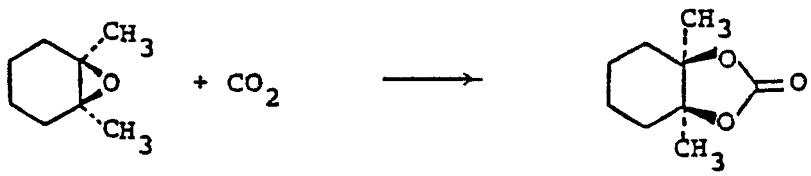

2. Stufe:

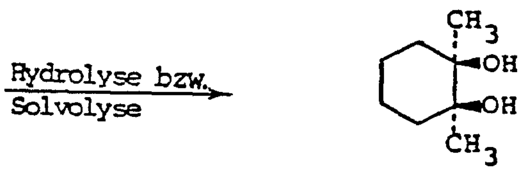


Die als Ausgangsprodukte zu verrwendenden cyclischen 1,2-Epoxide sind durch die Formel (II) allgemein definiert. In dieser Formel stehen $R^1$ und $R^2$, die gleich oder verschieden sein können, vorzugsweise für Wasserstoff, Alkyl mit 1—4 C-Atomen, Cycloalkyl mit 5—6 C-Atomen, Phenyl oder Benzyl; der Index n steht vorzugsweise für 1 oder 2; R steht vorzugsweise für Wasserstoff, Hydroxy, Halogen (insbesondere Fluor, Chlor oder Brom), Cyano, Alkyl mit 1—6 C-Atomen, Phenyl, Benzyl, Alkoxy mit 1—6 C-Atomen, Alkoxycarbonyl mit 2—6 C-Atomen, Halogenalkyl mit 1—2 C-Atomen, Phenoxy oder einen heterocyclischen Rest mit 5—6 Ringgliedern, der Sauerstoff, Schwefel und/oder Stickstoff als Heteroatome enthält, wobei im Falle n = 2 die zwei Reste R gleich oder verschieden sein können.

Die cyclischen 1,2-Epoxide der Formel (II) sind *cis*-Epoxide, soweit die Stereochemie des Epoxidringes im Verhältnis zu dem carbocyclischen Ring betroffen ist. Die übrigen Substituenten, außer an $C^1$ und $C^2$, können eine beliebige stereochemische Anordnung aufweisen. Bei den erfindungsgemäß einzusetzenden Epoxiden handelt es sich um Cyclopenten-(1,2)-oxid, Cyclohexen-(1,2)-oxid und Cyclohepten-(1,2)-oxid, sowie Substitutionsderivate dieser Stammverbindungen. Die oben definierten Substituenten $R^1$, $R^2$ bzw. $R_n$ sind lediglich beispielhaft angegeben; grundsätzlich können die genannten Stammverbindungen beliebig substituiert sein, sofern die Substituenten die Bedingung erfüllen, daß sie unter den Reaktionsbedingungen weder mit Kohlendioxid noch mit dem Hydrolyse- bzw. Solvolysemedium in Reaktion treten.

Die cyclischen 1,2-Epoxide (II) sind bekannt oder können nach bekannten Verfahren hergestellt werden (vgl. z.B. Houben-Weyl, Methoden der Organischen Chemie, 4. Auflage, Band VI/3, S. 371—434 (1965); ferner DE—OS 2 734 085 und DE—OS 2 734 086). Als Beispiele seien im einzelnen folgende Epoxide genannt:

Cyclopenten-(1,2)-oxid
3-Chlorcyclopenten-(1,2)-oxid
3,5-Dichlorcyclopenten-(1,2)-oxid
4-Hydroxycyclopenten-(1,2)-oxid
3,5-Dimethylcyclopenten-(1,2)-oxid
3,5-Diethylcyclopenten-(1,2-oxid
4-Isopropylcyclopenten-(1,2)-oxid
4-tert.-Butylcyclopenten-(1,2)-oxid
3,5-Diphenylcyclopenten-(1,2)-oxid
3,5-Di-(4-chlorphenyl)-cyclopenten-(1,2)-oxid
4-Phenylcyclopenten-(1,2)-oxid
3-Methoxycyclopenten-(1,2)-oxid
4-Propoxycyclopenten-(1,2)-oxid
3,5-Diisopropoxycyclopenten-(1,2)-oxid
4-tert.-Butoxycyclopenten-(1,2)-oxid
4-n-Hexoxycyclopenten-(1,2)-oxid
3-Carbomethoxycyclopenten-(1,2)-oxid
4-Carbopropoxycyclopenten-(1,2)-oxid
3,5-Di-[(β-Carbomethoxy)-ethyl]-cyclopenten-(1,2)-oxid
3-Cyancyclopenten-(1,2)-oxid

4-Cyancyclopenten-1,2-oxid
4-(β-Cyanethyl)-cyclopenten-(1,2)-oxid
3-Fluorcyclopenten-(1,2)-oxid
3-Trifluormethyl-cyclopenten-(1,2)-oxid
1,2-Dimethylcyclopenten-(1,2)-oxid
1-Methylcyclopenten-(1,2)-oxid
1-Phenylcyclopenten-(1,2)-oxid
1-Ethylcyclopenten-(1,2)-oxid
1-Propylcyclopenten-(1,2)-oxid
1,2-Diethylcyclopenten-(1,2)-oxid
1,2-Diphenylcyclopenten-(1,2)-oxid
Cyclohexen-(1,2)-oxid
3-trifluormethyl-cyclohexen-(1,2)-oxid
3-Chlorcyclohexen-(1,2)-oxid
4-Chlorcyclohexen-(1,2)-oxid
5-Chlorcyclohexen-(1,2)-oxid
4,5-Dichlorcyclohexen-(1,2)-oxid
3-Hydroxycyclohexen-(1,2)-oxid
3,5-Dihydroxycyclohexen-(1,2)-oxid
3-Methylcyclohexen-(1,2)-oxid
5-Ethylcyclohexen-(1,2)-oxid
3,5-Diisopropylcyclohexen-(1,2)-oxid
4,5-Di-n-hexylcyclohexen-(1,2)-oxid
4-Phenylcyclohexen-(1,2)-oxid
4,5-Diphenylcyclohexen-(1,2)-oxid
4-(p-Chlorphenyl)-cyclohexen-(1,2)-oxid
3-Methoxycyclohexen-(1,2)-oxid
4-Ethoxycyclohexen-(1,2)-oxid
5-Isopropoxycyclohexen-(1,2)-oxid
4-Hexoxycyclohexen-(1,2)-oxid
4-(β-Cyanethyl)-cyclohexen-(1,2)-oxid
4-(1,3-Dioxolan-2-yl)-cyclohexen-(1,2)-oxid
1-Methylcyclohexen-(1,2)-oxid
1-Ethylcyclohexen-(1,2)-oxid
1-Phenylcyclohexen-(1,2)-oxid
1-Propylcyclohexen-(1,2)-oxid
1,2-Dimethylcyclohexen-(1,2)-oxid
1,2-Diethylcyclohexen-(1,2)-oxid
1,2-Diphenylcyclohexen-(1,2)-oxid
Cyclohepten-(1,2)-oxid
3-Methylcyclohepten-(1,2)-oxid
3,7-Dimethylcyclohepten-(1,2)-oxid
4,5,6-Trimethylcyclohepten-(1,2)-oxid
5-Isopropylcyclohepten-(1,2)-oxid
5-tert.-Butylcyclohepten-(1,2)-oxid
3-Chlorcyclohepten-oxid
4-(β-Chlorethyl-)-cyclohepten-(1,2)-oxid
4,6-Dichlorcyclohepten-(1,2)-oxid
5-Hydroxycyclohepten-(1,2)-oxid
4,5-Dihydroxycyclohepten-(1,2)-oxid
3-Phenylcyclohepten-(1,2)-oxid
5-Phenylcyclohepten-(1,2)-oxid
4,6-Di-[(p-tert.-butyl)-phenyl]-cyclohepten-(1,2)-oxid
3-Methoxycyclohepten-(1,2)-oxid
5-Methoxycyclohepten-(1,2)-oxid
3-Propoxycyclohepten-(1,2)-oxid
5-tert.-Butoxycyclohepten-(1,2)-oxid
3-Carbomethoxycyclohepten-(1,2)-oxid
4-Carbomethoxycyclohepten-(1,2)-oxid
3,7-Dicarbomethoxycyclohepten-(1,2)-oxid
5-(β-Carbomethoxy)-ethyl-cyclohepten-(1,2)-oxid
1-Methylcyclohepten-(1,2)-oxid
1,2-Dimethylcyclohepten-(1,2)-oxid
1-Phenylcyclohepten-(1,2)-oxid
1-Ethylcyclohepten-(1,2)-oxid

1-Propylcyclohepten-(1,2)-oxid
1,2-Diethylcyclohepten-(1,2)-oxid
1,2-Diphenylcyclohepten-(1,2)-oxid

Die Umsetzung der cyclischen 1,2-Epoxide (II) mit Kohlendioxid ($CO_2$) wird bei einem Druck von 20—200 bar, vorzugsweise bei 30—100 bar, besonders bevorzugt bei 30—50 bar durchgeführt. Die Reaktionstemperatur liegen zwischen 100 und 300°C, vorzugsweise zwischen 130 und 230°C.

Als Carbonierungskatalysator kommen die üblichen Alkylenoxidcarbonierungskatalysatoren in Frage, wie z.B. Ammoniumsalze, Phosphoniumsalze, Alkali- und Erdalkali-oxide, carbonate und -halogenide, vorzugsweise jedoch Ammoniumsalze, besonders bevorzugt Tetraalkylammoniumhalogenide, wie beispielsweise Tetraethylammoniumbromid und Tetrabutylammoniumbromid (vgl. hierzu z.B. DE—OS 2 855 232, DE—AS 1 169 459, DE—AS 1 135 490).

Der Carbonierungskatalysator wird im allgemeinen in Menge von 0,001—0,1 Mol, vorzugsweise von 0,001—0,05 Mol, pro Mol Epoxid (II) eingesetzt.

Die Hydrolyse der reinen cyclischen 1,2-*cis*-Carbonate (III) wird mit Wasser, einer Lösung eines Salzes in Wasser oder mit verdünnter wäßriger Säure oder Base durchgeführt. Die Konzentration der Säuren bzw. Basen liegt im allgemeinen zwischen 0,01% und 10%, vorzugsweise zwischen 0,1% und 3%. Die Reaktonstemperaturen für die Hydrolyse liegen im allgemeinen zwischen 80 und 200°C, vorzugsweise zwischen 100 und 120°C, wobei gegebenenfalls in einem Druckgefäß gearbeitet werden muß, in welchem sich in Abhängigkeit von der jeweiligen Zusammensetzung des Reaktionsgemsiches und von der gewählten Temperatur jeweils ein bestimmter Überdruck einstellt. Das Hydrolysegemisch wird so lange erhitzt, bis kein Carbonat (III) mehr nachzuweisen ist.

Geeignete Säuren sind die anorganischen Mineralsäuren in verdünnter wäßriger Lösung, vorzugsweise Salzsäure und Schwefelsäure. Geeignete Basen sind insbesondere die Alkalihydroxide in wäßriger Lösung, bevorzugt Natronlauge und Kalilauge.

Die Carbonat (III) können auch mit der Lösung eines Alkoholats in einem Alkohol solvolysiert werden, wobei man bei Temperaturen zwischen 50 und 100°C arbeitet. Zweckmäßigerweise verwendet man alkoholische Lösungen mit einem Alkoholatgehalt von 0,1—30 Gewichtsprozent. Besonders geeignet sind z.B. Natriummethylat in Methanol und Natriumethylat in Ethanol.

Gegebenenfalls kann die Hydrolyse bzw. Solvolyse auch ohne Reinigung der Carbonat (III) vorgenommen werden, wobei nicht eingesetzte Mengen an Epoxid (II) bei vermindertem Druck abdestilliert werden und man das zurückbleibende Carbonierungsgemisch der oben beschriebenen Hydrolyse bzw. Solvolyse unterwirft.

Es ist ebenfalls möglich, die gesamte Masse des Carbonierungsgemisches der Hydrolyse oder Solvolyse zu unterwerfen, wobei in Kauf genommen wird, daß als Hauptprodukt entstehende *cis*-Diol (I) mit einer dem nicht umgesetzten Epoxid (II) entsprechenden Menge des mit (I) isomeren *trans*-Diols verunreinigt ist.

Bei den beiden zuletzt beschriebenen Ausführungsformen wird im Effekt mit wäßrigen Salzlösungen hydrolysiert, wenn man die Reaktionsgemische mit Wasser erhitzt, da der salzförmige Carbonierungskatalysator bei Zusatz von Wasser eine entsprechende Salzlösung ergibt.

Die Hydrolyse- bzw. Solvolysegemische werden nach beendeter Reaktion zweckmäßigerweise neutralisiert und durch Destillation aufgearbeitet, sofern eine Reinisolierung der Diole (I) gewünscht wird.

Die erfindungsgemäß herstellbaren cyclischen 1,2-*cis*-Diole (I) sind wertvolle Zwischenprodukte für die Synthese von bestimmten herbiziden Wirkstoffen (vgl. z.B. DE—OS 2 753 556, DE—OS 2 842 188, US—PS 4 282 388).

## Beispiele

### Beispiele 1

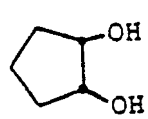

1. Stufe-Carbonat:

500 g (5,95 Mol) Cyclopentenoxid und 4 g (0,019 Mol) Tetraethylammoniumbromid werden in einen mit Kohlendioxid gespülten Rührautoklaven gefüllt. Es wird auf 180°C erhitzt und eine solche Menge Kohlendioxid aufgepreßt, daß sich ein Druck von 40 bar einstellt. Der Druck wird durch Nachpressen von Kohlendioxid auf diesem Wert gehalten, bis keine Druckabnahme mehr zu beobachten ist. Der Autoklav wird abgekühlt, entspannt und das Produkt durch Destillation im Vakuum gereinigt. Man erhält 703 g (≙ 92% der Theorie) 1,2-*cis*-Cyclopentencarbonat; Siedepunkt 142°C/0,2 mbar.

Der Vorlauf enthält nicht umgesetztes Cyclopentenoxid.

2. Stufe-Diol:

500 g (3,90 Mol) des so gewonnenen 1,2-*cis*-Cyclopentencarbonats werden mit 1 Liter 1-%iger Natronlauge 2-Stunden unter Rückfluß erhitzt. Nach gaschromatografischer Analyse ist die Ausgangsverbindung vollständig verschwunden zugunsten einer einzigen neuen Substanz, bei der es sich um 1,2-

*cis*-Cyclopentandiol handelt (≙ 100% der Theorie) wie durch unabhängige Synthese gezeigt werden konnte. Man neutralisiert mit 10%iger Salzsäure und reinigt durch Destillation im Vakuum. Man erhält 370 g (≙ 93% der Theorie) reines 1,2-*cis*-Cyclopentandiol; Siedepunkt 106°C/16 mbar.

Beispiel 2
(Variante von Beispiel 1)

500 g (5,95 Mol) Cyclopentenoxid werden wie in Beispiel 1 (1. Stufe) beschrieben mit Kohlendioxid zum Carbonat umgesetzt. Nach dem Abkühlen und Entspannen des Autoklaven wird die gesamte Mischung im Vakuum "andestilliert", bis kein nicht umgesetztes Cyclopentenoxid mehr übergeht. Der zurückbleibende Rest der Mischung wird in die Hydrolyse eingesetzt; dann wird wie unter Beispiel 1 (2. Stufe) Verfahren. Man erhält reines 1,2-*cis*-Cyclopentandiol vom Siedepunkt 106°C/16 mbar.

Beispiel 3
(Variante von Beispielen 1 und 2)

1 kg (11,9 Mol) Cyclopentenoxid und 10 g (0,031 Mol) Tetrabutylammoniumbromid werden in einen mit Kohlendioxid gespülten Rührautoklaven gefüllt. Es wird auf 190°C erhitzt und eine solche Menge Kohlendioxid aufgepreßt, daß sich ein Druck von 45 bar einstellt. Der Druck wird durch Nachpressen von Kohlendioxid auf diesem Wert gehalten, bis keine weitere Druckabnahme mehr zu beobachten ist. Der Autoklav wird anschließend abgekühlt und entspannt; dann wird 1 Liter 2-%ige Kalilauge zugeleitet und 2 Stunden unter Rückflußt erhitzt. Man stellt mit 25%iger Schwefelsäure neutral und destilliert im Vakuum. Das so erhaltene 1,2-*cis*-Cyclopentandiol (Seidepunkt 106°C/16 mbar) ist mit 5—10% 1,2-*trans*-Cyclopentandiol verunreinigt.

Beispiel 4

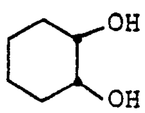

1. Stufe-Carbonat:

500 g (5, 10 Mol) Cyclohexenoxid und 5 g (0,024 Mol) Tetraethylammoniumbromid werden in einen mit Kohlendioxid gespülten Rührautoklaven gefüllt. Es wird auf 180°C erhitzt und eine solche Mengen Kohlendioxid aufgepreßt, daß sich ein Druck von 40 bar einstellt. Dieser Druck wird durch Nachpressen von Kohlendioxid aufrechterhalten, bis keine Druckabnahme mehr zu beobachten ist. Der Autoklav wird abgekühlt und entspannt. Das entstandene 1,2-*cis*-Cyclohexencarbonat wird durch Destillation gereinigt; Ausbeute 680 g (≙ 94% der Theorie); Siedepunkt 105—109°C/0,7 mbar). Das Produkt ist identisch mit einer auf unabhängigem Weg synthetisierten Vergleichsprobe.

2. Stufe-Diol:

500 g (3,52 Mol) des so hergestellten Carbonats werden mit 500 ml 0,5%iger Natronlauge 2 Stunden zum Sieden erhitzt. Das Hydrolysat enthält nach Kapillar-Gaschromatogramm reines 1,2-*cis*-Cyclohexandiol, das durch Destillation isoliert wird. Ausbeute 372 g (≙ 91% der Theorie); Siedepunkt 117°C/16 mbar.

Beispiel 5

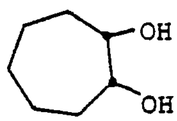

1. Stufe-Carbonat:

500 g (4,46 Mol) Cycloheptenoxid und 5 g (0,024 Mol) Tetraethylammoniumbromid werden in einem mit Kohlendioxid gespülten Rührautoklaven auf 130°C erhitzt und es wird solange Kohlendioxid aufgepreßt, bis sich ein Druck von 50 bar einstellt. Der Druck wird durch Nachpressen von Kohlendioxid auf diesem Wert gehalten, bis keine Abnahme mehr zu beobachten ist. Der Autoklav wird abgekühlt und entspannt und das Produkt im Vakuum gereinigt. Man erhält 627 g (≙ 90% der Theorie) 1,2-*cis*-Cycloheptencarbonat (Siedepunkt: 165°C/15 mbar; Schmelzpunkt: 70°C), welches mit einem aus 1,2-*cis*-Cycloheptandiol und Phosgen hergestellten Produkt identisch ist.

2. Stufe-Diol:

1 kg (6,41 Mol) des so hergestellten Cycloheptencarbonats werden mit 2 Liter 1-%-Natronlauge 2 Stunden unter Rückfluß erhitzt. Im Kapillar-Gaschromatogramm zeigt sich, daß die Ausgangsverbindung verschwunden ist und sich *ein* neues Produkt gebildet hat, bei dem es sich um 1,2-*cis*-Cycloheptandiol handelt, wie durch unabhängige Synthese gezeigt werden konnte. Man stellt neutral und reinigt durch Destillation im Vakuum. Man erhält 775 g (≙ 93% der Theorie) reines 1,2-*cis*-Cycloheptandiol; Siedepunkt: 132°C/16 mbar; Schmelzpunkt 49°C.

Beispiel 6

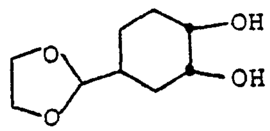

1. Stufe-Carbonat:

500 g (2,94 Mol) 4-(1,3-Dioxolan-2-yl)-cyclohexen-(1,2)-oxid und 6 g (0,029 Mol) Tetraethylammoniumbromid werden in einen mit Kohlendioxid gespülten Rührautoklaven gefüllt. Es wird auf 130°C erhitzt und Kohlendioxid aufgepreßt, bis sich ein Druck von 40 bar einstellt. Dieser Druck wird durch Nachpressen von Kohlendioxid aufrechterhalten, bis keine Abnahme mehr zu beobachten ist. Der Autoklav wird abgekühlt, entspannt und das Produkt im Vakuum gerinigt. Man erhält 616 g (≙ 98% der Theorie) Carbonat (*cis* bezüglich der C-Atome 1 und 2, *cis/trans*-Gemisch bezüglich der C-Atome 1/2 und 4; das Isomerenverhältnis entspricht dem des eingesetzten Epoxids); Siedepunkt; 175°C/0,15 mbar.

Zur Herstellung des Ausgangsproduktes vergl. Chem. Abstr. *73,* 25065v (1970).

2. Stufe-Diol:

300 g (1,40 Mol) des so gewonnenen *cis*-Carbonats werden in 500 ml 2%iger Natronlauge 2 Stunden unter Rückfluß erhitzt. Man erhält quantitativ eine Mischung der beiden *cis*-1,2-Diole (*cis* bezüglich der OH-Gruppen an den C-Atomen 1 und 2; *cis/trans* bezüglich des Dioxolanrestes an C-Atom 4; das Isomerenverhältnis entspricht dem des eingesetzten Carbonats aus der 1. Stufe). Das Produkt wird nach Neutralisation mit 10%iger Salzsäure durch Destillation rein isoliert; Ausbeute: 250 g(≙ 95% der Theorie) 4-(1,3-Dioxolan-2-yl)-cyclohexan-1,2-diol; Siedepunkt: 134°C/0,3 mbar.

## Patentansprüche

1. Verfahren zur Herstellung von cyclischen 1,2-*cis*-Diolen der allgemeinen Formel I

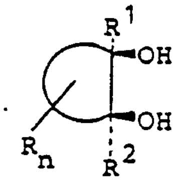

(I)

worin der carbocyclische Ring 5—7 Kohlenstoffatome enthält und

$R^1$ und $R^2$ gleich oder verschieden sind und unabhängig voneinander für Wasserstoff oder gegebenenfalls substituierte Alkyl-, Cycloalkyl-, Alkenyl-, Aryl- oder Aralkylreste stehen,

n für 1, 2, 3 oder 4 steht und

R für Wasserstoff, Hydroxy, Halogen, Cyano oder gegebenenfalls substituierte Alkyl-, Cycloalkyl-, Alkenyl-, Aryl-, Aralkyl-, Alkoxy-, Alkoxycarbonyl-, Aryloxy-, Alkylthio-, Arylthio- oder heterocyclische Reste steht, wobei für den Fall n >1 die Reste R gleich oder verschieden sein können und wobei 2 benachbarte Reste R, gegebenenfalls auch zweimal, für eine Doppelbindung stehen können, dadurch gekennzeichnet, daß man cyclische 1,2-Epoxide der allgemeinen Formel II

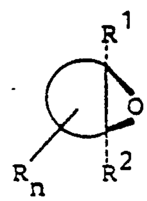

(II)

worin der carbocyclische Ring 5—7 Kohlenstoffatome enthält und $R^1$, $R^2$, n und R die oben angegebene Bedeutung haben,

mit Kohlendioxid in Gegenwart eines Carbonierungskatalysators bei Drucken von 20—200 bar und bei Temperaturen zwischen 100 und 300°C umsetzt, woebi der Druck durch Nachpressen von Kohlendioxid solange konstant gehalten wird, bis keine Druckabnahme mehr beobachtet wird, — (1. Stufe) — und die hierbei gebildeten cyclischen 1,2-*cis*-Carbonate der allgemeinen Formel (III)

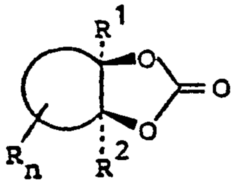

(III)

worin der carbocyclische Ring wiederum 5—7 Kohlenstoffatome enthält und $R^1$, $R^2$, n und R die oben angegebene Bedeutung haben,

mit Wasser, wäßrigen Salzlösungen, verdünnten wäßrigen Säuren oder Basen hydrolysiert oder mit der Lösung eines Alkoholats in einem Alkohol solvolysiert, jeweils bei erhöhter Temperatur und gegebenenfalls unter erhöhtem Druck (2. Stufe).

2. Verfahren nach Anspruch 1, dadurch gekennzeichnet, daß die Umsetzung der Epoxide (II) mit Kohlendioxid (1. Stufe) bei einem Druck von 30—100 bar durchgeführt wird.

3. Verfahren nach Anspruch 1, dadurch gekennzeichnet, daß die Umsetzung der Epoxide (II) mit Kohlendioxid (1. Stufe) bei Temperaturen zwischen 130 und 230°C, durchgeführt wird.

4. Verfahren nach Anspruch 1, dadurch gekennzeichnet, daß bei der 1. Stufe des Verfahrens als Carbonierungskatalysator ein üblicher Alkylenoxidcarbonierungskatalysator aus der Gruppe der Ammoniumsalze, Phosphoniumsalze, Alkali- und Erdalkali-oxide, -carbonate und -halogenide, vorzugsweise ein Ammoniumsalz, besonders bevorzugt ein Tetraalkylammoniumhalogenid wie Tetraethylammoniumbromid oder Tetrabutylammoniumbromid, eingesetzt wird.

5. Verfahren nach Anspruch 1, dadurch gekennzeichnet, daß die Hydrolyse der Carbonate (III) (2. Stufe) mit Wasser, wäßrigen Salzlösungen oder verdünnten wäßrigen Säuren oder Basen bei Temperaturen zwischen 80 und 200°C, vorzugsweise zwischen 100 und 120°C, durchgeführt wird, und daß die Solvolyse der Carbonate (III) mit der Lösung eines Alkoholats in einem Alkohol bei Temperaturen zwischen 50 und 100°C durchgeführt wird, wobei gegebenenfalls in einem Druckgefäß gearbeitet werden muß, in welchem sich in Abhängigkeit von der jeweiligen Zusammensetzung des Reaktionsgemsiches und von der gewählten Temperatur jeweils ein bestimmter Überdruck einstellt.

6. Verfahren nach Anspruch 1, dadurch gekennzeichnet, daß bei der 2. Stufe des Verfahrens die wäßrigen Salzlösungen oder die verdünnten wäßrigen Säuren oder Basen jeweils in Form von 0,01—10%-igen Lösungen, vorzugsweise von 0,1—3%-igen Lösungen eingesetzt werden.

7. Verfahren nach Anspruch 1, dadurch gekennzeichnet, daß in den Epoxiden der Formel (II) bzw. Carbonaten der Formel (III) jeweils

$R^1$ und $R^2$ gleich oder verschieden sind und unabhängig voneinander für Wasserstoff, Alkyl mit 1—4 C-Atomen, Cycloalkyl mit 5—6 C-Atomen, Phenyl oder Benzyl steht,

n für 1 oder 2 steht und

R für Wasserstoff, Hydroxy, Halogen, (insbesondere Fluor, Chlor oder Brom), Cyano, Benzyl, Alkoxy mit 1—6 C-Atomen, Alkoxycarbonyl mit 2—6 C-Atomen, Halogenalkyl mit 1—2 C-Atomen, Phenoxy oder einen heterocyclischen Rest mit 5—6 Ringgliedern, der Sauerstoff, Schwefel und/oder Stickstoff als Heteroatome enthält, wobei im Falle n = 2 die 2 Reste gleich oder verschieden sein können.

8. Verfahren nach Anspruch 1, dadurch gekennzeichnet, daß als Epoxide (II) Cyclopentenoxid, Cyclohexenoxid oder Cycloheptenoxid eingesetzt werden, aus welchen man über die entsprechenden *cis*-Carbonate *cis*-Cyclopentan-1,2-diol, *cis*-Cyclohexan-1,2-diol bzw. *cis*-Cycloheptan-1,2-diol erhält.

9. Verfahren zur Herstellung von cyclischen 1,2-*cis*-Carbonaten der allgemeinen Formel III

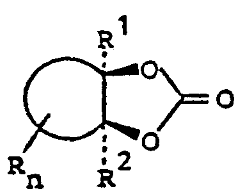

(III)

worin der carbocyclische Ring 5—7 Kohlenstoffatome enthält und

$R^1$, $R^2$, n und R die in Anspruch 1 angegebene Bedeutung haben, dadurch gekennzeichnet, daß man cyclische 1,2-Epoxide der allgemeinen Formel II

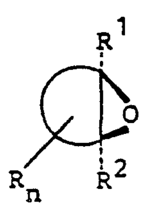

(II)

worin der carbocyclische Ring 5—7 Kohlenstoffatome enthält und $R^1$, $R^2$, n und R die angegeben Bedeutung haben, mit Kohlendioxid in Gegenwart eines Carbonierungskatalysators unter erhöhtem Druck und bei erhöhter Temperatur umsetzt.

**Revendications**

1. Procédé de production de 1,2-*cis*-diols cycliques de formule générale I

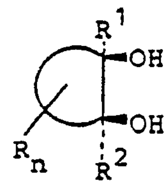

(I)

dans laquelle le noyau carbocyclique contient 5 à 7 atomes de carbone et

$R^1$ et $R^2$ sont identiques ou différents et représentent, indépendamment l'un de l'autre, l'hydrogène ou un reste alkyle, cycloalkyle, alcényle, aryle ou aralkyle éventuellement substitué,

*n* a la valeur 1, 2, 3 ou 4 et

R représente l'hydrogène, un groupe hydroxy, un halogène, un groupe cyano ou des restes alkyle, cycloalkyle, alcényle, aryle, aralkyle, alkoxy, alkoxycarbonyle, aryloxy, alkylthio, arylthio ou hétérocyclique éventuellement substitues, les restes R pouvant être identiques ou différents pou le cas où *n* est supérieur à 1 et deux restes R voisins pouvant dans ce cas former une double liaison, deux fois, le cas échéant, caractérisé en ce qu'on fait réagir des 1,2-époxydes cycliques de formule générale II

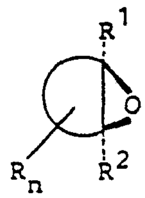

(II)

dans laquelle le noyau carbocyclique contient 5 à 7 atomes de carbone et $R^1$, $R^2$, *n* et R ont la définition indiquée ci-dessous,

avec l'anhydride carbonique en présence d'un catalyseur de carbonation à des pressions de 20 à 200 bars et à des températures comprises entre 100 et 300°C, la pression étant maintenue constante par poursuite de l'introduction d'anhydride carbonique sous pression jusqu'à ce qu'on n-observe plus de chute de pression (première étape), et on hydrolyse les 1,2-*cis*-carbonates cycliques ainsi formés, de formule générale III

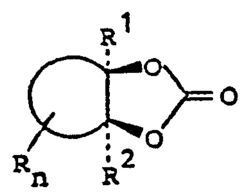

(III)

dans laquelle le noyau carbocyclique contient la encore 5 à 7 atomes de carbone et $R^1$, $R^2$, *n* et R ont la définition indiquée ci-dessus,

avec de l'eau, des solutions aqueuses de sels, des acides ou des bases aqueux dilués ou on les solvolyse avec la solution d'un alcoolate dans un alcool, dans les deux cas à température élevée et, le cas échéant, sous pression élevée (seconde étape).

2. Procédé suivant la revendication 1, caractérisé en ce qu'on conduit la réaction des époxydes (II) avec l'anhydride carbonique (première étape) à une pression de 30 à 100 bars.

3. Procédé suivant la revendication 1, caractérisé en ce qu'on conduit la réaction des époxydes (II) avec l'anhydride carbonique (première étape) à des températures comprises entre 130 et 230°C.

4. Procédé suivant la revendication 1, caractérisé en ce qu'on utilise dans sa première étape comme catalyseur de carbonatation un catalyseur classique de carbonatation d'oxydes alkyléniques choisi dans le groupe des sels d'ammonium, des sels de phosphonium, des oxydes, carbonates et halogénures alcalins et alcalino-terreux, de préférence un sel d'ammonium, tout particulièrement un halogénure de tétra-alkylammonium tel que le bromure de tétra-éthylammonium ou le bromure de tétrabutylammonium.

5. Procédé suivant la revendication 1, caractérisé en ce que l'hydrolyse des carbonates (III) (seconde

étape) est effectuée avec de l'eau, des, solutions aqueuses de sels ou des acides ou des bases aqueux dilués à des températures comprises entre 80 et 200°C, de préférence entre 100 et 120°C, et en ce qu'on effectue la solvolyse des carbonates (III) avec une solution d'un alcoolate dans un alcool a des temperatures comprises entre 50 et 100°C, et on doit alors opérer, le cas échéant, dans un récipient résistant à la pression dans lequel une surpression déterminée s'établit dans chaque cas en fonction de la composition existante du mélange réactionnel et de la température choisie.

6. Procédé suivant la revendication 1, caractérisé en ce qu'on utilise dans sa seconde étape les solutions aqueuses de sels ou les acides ou les bases aqueux dilués dans chaque cas sous la forme de solutions à 0,01—10%, de préférence de solutions à 0,1—3%.

7. Procédé suivant la revendication 1, caractérisé en ce que dans les époxydes de formule (II) et, respectivement, dans les carbonates de formule (III),

$R^1$ et $R^2$ sont identiques ou différents et représentent, indépendamment l'un de l'autre, l'hydrogène, des groupes alkyle ayant 1 à 4 atomes de carbone, cycloalkyle ayant 5 ou 6 atomes de carbone, le groupe phényle ou le groupe benzyle,

*n* a la valeur 1 ou 2 et

R représente l'hydrogène, un groupe hydroxy, un halogène (notamment fluor, chlore ou brome), un groupe cyano, benzyle, alkoxy ayant 1 à 6 atomes de carbone, alkoxycarbonyl ayant 2 à 6 atomes de carbone, halogénalkyle ayant 1 ou 2 atomes de carbone, phénoxy ou un reste hétérocyclique pentagonal ou hexagonal qui contient comme hétéroatomes des atomes d'oxygène, de soufre et/ou d'azote, et les deux restes R peuvent être identiques ou différents au cas où *n* est égal à 2.

8. Procédé suivant la revendication 1, caractérisé en ce qu'on utilise comme époxydes (II) le cyclopentène-oxyde, le cyclohexène-oxyde ou le cycloheptène-oxyde, à partir desquels on obtient le *cis*-cyclopentane-1,2-diol, le *cis*-cyclohexane-1,2-diol et, respectivement, le *cis*-cycloheptane-1,2-diol en passant par les *cis*-carbonates correspondants.

9. Procédé de production de 1,2-*cis*-carbonates cycliques de formule générale III

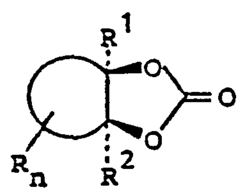

(III)

dans laquelle le noyau carbocyclique contient 5 à 7 atomes de carbone et

$R^1$, $R^2$, *n* et R ont la définition indiquée dans la revendication 1, caractérisé en ce qu'on fait réagir des 1,2-époxydes cycliques de formule générale II

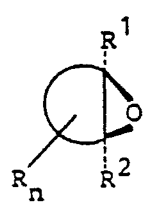

(II)

dans laquelle le noyau carbocyclique contient 5 à 7 atomes de carbone et $R^1$, $R^2$, *n* et R ont la définition indiquée,

avec l'anhydride carbonique en présence d'un catalyseur de carbonation sous pression élevée et à température élevée.

**Claims**

1. Process for the preparation of cyclic 1,2-*cis*-diols of the general formula I

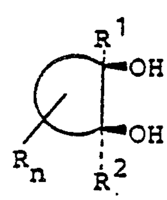

(I)

wherein the carbocyclic ring contains 5—7 carbon atoms and

$R^1$ and $R^2$ are identical or different and independently of each other represent hydrogen or optionally substituted alkyl, cycloalkyl, alkenyl, aryl or aralkyl radicals,

n represents 1, 2, 3 or 4 and

R represents hydrogen, hydroxyl, halogen, cyano or optionally substituted alkyl cycloalkyl, alkenyl, aryl, aralkyl, alkoxy, alkoxycarbonyl, aryloxy, alkylthio, arylthio or heterocyclic radicals, it being possible, in the case n >1, for the radicals R to be identical or different, and it being possible for 2 adjacent radicals R, optionally also twice, to represent a double bond, characterised in that cyclic 1,2-epoxides of the general formula II

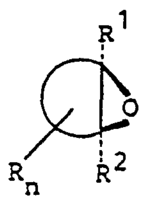

(II)

wherein the carbocyclic ring contains 5—7 carbon atoms and

$R^1$, $R^2$, n and R have the abovementioned meaning, are reacted with carbon dioxide in the presence of a carbonation catalyst under pressures of 20—200 bars and at temperatures between 100 and 300°C, the pressure being kept constant by pumping in further carbon dioxide until a decrease in pressure is no longer observed, — (1st stage) — and the cyclic 1,2-*cis*-carbonates formed in this reaction, of the general formula (III)

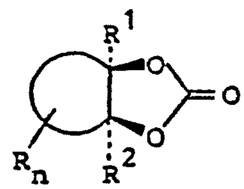

(III)

wherein the carbocyclic ring again contains 5—7 carbon atoms and

$R^1$, $R^2$, n and R have the abovementioned meaning, are hydrolysed with water, aqueous salt solutions, dilute aqueous acids or bases or solvolysed with a solution of an alcoholate in an alcohol, in each case at an elevated temperature, and if appropriate, under an elevated pressure (2nd stage).

2. Process according to Claim 1, characterised in that the reaction of the epoxides (II) with carbon dioxide (1st stage) is carried out under a pressure of 30—100 bars.

3. Process according to Claim 1, characterised in that the reaction of the epoxides (II) with carbon dioxide (1st stage) is carried out at temperatures between 130 and 230°C.

4. Process according to Claim 1, characterised in that a customary alkylene oxide carbonation catalyst from the group comprising the ammonium salts, phosphoniuim salts, alkali metal and alkaline earth metal oxides, carbonates and halides, preferably an ammonium salt, and paticularly preferably a tetraalkylammonium halide such as tetraethylammonium bromide or tetrabutylammonium bromide, is used as the carbonation catalyst in the 1st stage of the process.

5. Process according to Claim 1, characterised in that the hydrolysis of the carbonates (III) (2nd stage) is carried out with water, aqueous salt solutions or dilute aqueous acids or bases at temperatures between 80 and 200°C, preferably between 100 and 120°C and in that the solvolysis of the carbonates (III) is carried out with the solution of an alcoholate in an alcohol at temperatures between 50 and 100°C, it possibly being necessary to carry out the reaction in a pressure vessel in which a specific excess pressure establishes itself in each case according to the particular composition of the reaction mixture and the temperature selected.

6. Process according to Claim 1, characterised in that, in the 2nd stage of the process, the aqueous salt solutions or the dilute aqueous acids or bases are in each case used in the form of 0.01—10% strength solutions, preferably 0.1—3% strength solutions.

7. Process according to Claim 1, characterised in that, in the epoxides of the formla (II) or carbonates of the formula (III),

$R^1$ and $R^2$ are, in each case, identical or different and independently of each other represent hydrogen, alkyl with 1—4 C atoms, cycloalkyl with 5—6 C atoms, phenyl or benzyl,

n in each case represents 1 or 2 and

R in each case represents hydrogen, hydroxyl, halogen, (in particular fluorine, chlorine or bromine), cyano, benzyl, alkoxy with 1—6 C atoms, alkoxycarbonyl with 2—6 C atoms, halogenoalkyl with 1—2 C atoms, phenoxy or a heterocyclic radical with 5—6 ring members which contains oxygen, sulphur and/or nitrogen at hetero atoms, wherein in the case where n = 2 the 2 radicals R can be identical or different.

8. Process according to Claim 1, characterised in that cyclopentene oxide, cyclohexene oxide or cycloheptene oxide, from which *cis*-cyclopentane-1,2-diol, *cis*-cyclohexane-1,2-diol or *cis*-cycloheptane-1,2-diol are obtained via the corresponding *cis*-carbonates, are used as the epoxides (II).

9. Process for the preparation of cyclic 1,2-*cis*-carbonates of the general formula III

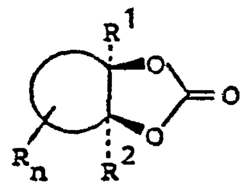

(III)

wherein the carbocyclic ring contains 5—7 carbon atoms and

$R^1$, $R^2$, n and R have the meaning given in Claim 1, characterised in that cyclic 1,2-epoxides of the general formula II

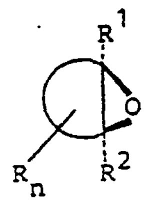

(II)

wherein the carbocyclic ring contains 5—7 carbon atoms and

$R^1$, $R^2$, n and R have the abovementioned meaning, are reacted with carbon dioxide in the presence of a carbonation catalyst under an elevated pressure and at an elevated temperature.